(19) Europäisches Patentamt
European Patent Office
Office européen des brevets

(11) Veröffentlichungsnummer: **0 527 314 B1**

(12) # EUROPÄISCHE PATENTSCHRIFT

(45) Veröffentlichungstag der Patentschrift: **07.09.94**

(51) Int. Cl.[5]: **C07D 249/06**, C07C 251/76

(21) Anmeldenummer: **92110766.0**

(22) Anmeldetag: **26.06.92**

(54) **Verfahren zur Herstellung von 2-Aryl-2H-1,2,3-triazolen.**

(30) Priorität: **28.06.91 CH 1920/91**

(43) Veröffentlichungstag der Anmeldung:
**17.02.93 Patentblatt 93/07**

(45) Bekanntmachung des Hinweises auf die Patenterteilung:
**07.09.94 Patentblatt 94/36**

(84) Benannte Vertragsstaaten:
**AT BE CH DE DK FR GB IT LI LU NL SE**

(56) Entgegenhaltungen:
**EP-A- 0 350 237**
**DE-A- 3 106 598**
**GB-A- 2 047 250**

**CHEMICAL ABSTRACTS, vol. 103, no. 19, 11. November 1985, Columbus, Ohio, US; abstract no. 160228d, Seite 680 ;**

(73) Patentinhaber: **LONZA AG**
**CH-3945**
**Gampel/Wallis (CH)**

(72) Erfinder: **Meul, Thomas, Dr.**
**Meisenweg 1**
**CH-Visp (Kanton Wallis) (CH)**

(74) Vertreter: **Weinhold, Peter, Dr. et al**
**Patentanwälte Dipl.-Ing. G. Dannenberg**
**Dr. P. Weinhold**
**Dr. D. Gudel**
**Dipl.-Ing. S. Schubert**
**Dr. P. Barz**
**Siegfriedstrasse 8**
**D-80803 München (DE)**

EP 0 527 314 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

2-Aryl-2H-1,2,3-triazole, insbesondere solche, in denen der Triazolring einen Substituenten in der Position 4 trägt, besitzen insektizide und acarizide Wirkung (EP-A 350 237).

Ein bekanntes Verfahren zu ihrer Herstellung geht von Arylhydrazonen von α-Hydroxyiminoaldehyden oder -ketonen aus, welche mit Oxidationsmitteln wie beispielsweise Kupfersulfat cyclisiert werden. Das so erhaltene N-oxid kann auf bekannte Weise reduziert werden. Die benötigten Hydroxyimino-arylhydrazone können durch Umsetzung des entsprechenden Arylhydrazins mit der entsprechenden α-Dicarbonylverbindung und Oximierung des so erhaltenen α-Carbonyl-arylhydrazons hergestellt werden. Das Verfahren ist umständlich und weist wegen der Oxidations- und Reduktionsschritte im Laufe der Synthese und bei der Herstellung des als Ausgangsmaterial benötigten Arylhydrazins weitere Nachteile, insbesondere eine grosse Abfallmenge, auf.

Wenn das Hydroxyimino-arylhydrazon zuerst acyliert wird, kann das so erhaltene Acyloxyimino-arylhydrazon auch durch Basenzusatz direkt in das gewünschte Triazol übergeführt werden, so dass die Bildung des N-oxids und dessen Reduktion vermieden werden. Stattdessen fällt jedoch ein Salz der der Acylgruppe entsprechenden Carbonsäure mit der zur Cyclisierung verwendeten Base als Abfall an.

Aufgabe der vorliegenden Erfindung war daher, für 4-substituierte 2-Aryl-2H-1,2,3-triazole der allgemeinen Formel

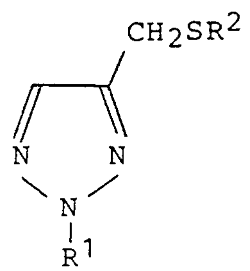

I

worin $R^1$ eine gegebenenfalls substituierte Aryl- oder Heteroarylgruppe und $R^2$ eine gegebenenfalls verzweigte und/oder substituierte Alkyl-, Cycloalkyl-, Arylalkyl- oder Arylgruppe bedeuten, ein einfaches und kostengünstiges Syntheseverfahren mit guter Ausbeute und geringen Abfallmengen bereitzustellen.

Erfindungsgemäss wird die Aufgabe durch das Verfahren nach Patentanspruch 1 gelöst.

Es wurde gefunden, dass das aus Diketen und Chlor auf an sich bekannte Weise (CH-PS 642 611) erhältliche γ-Chloracetoacetylchloridmit Aryldiazoniumsalzen direkt im Sinne einer Japp-Klingemann-Reaktion unter Erhalt der Chlormethylgruppe in die entsprechenden 3-Chlorpyruvaldehyd-arylhydrazone übergeführt werden kann.

Es war bekannt, dass die durch Hydrolyse aus dem Säurechlorid oder entsprechenden Estern erhältliche γ-Chloracetessigsäure in Gegenwart einer Base mit diazotierten Halogenanilinen zu 3-Chlorpyruvaldehyd-halophenylhydrazonen umgesetzt werden kann (DE-OS 30 12 193). Die γ-Chloracetessigsäure ist jedoch als β-Ketocarbonsäure wenig stabil und decarboxyliert leicht zu Chloraceton. Das erfindungsgemässe Verfahren kommt dagegen ohne Zusatz einer Base aus und erfordert keinen gesonderten Hydrolyseschritt. Damit entfällt auch die Gefahr einer Zersetzung der γ-Chloracetessigsäure.

Als Aryldiazoniumsalze können hierbei grundsätzlich alle durch Diazotierung von unsubstituierten oder substituierten primären mono- oder polycyclischen aromatischen Aminen nach bekannten Methoden (s. z.B. "Methoden der Organischen Chemie" (Houben-Weyl), 4. Auflage, Bd. X/3, S.12-64, Thieme Verlag, Stuttgart 1965) erhältlichen Diazoniumsalze verwendet werden. Vorzugsweise wird die Diazotierung mit Nitrosylschwefelsäure durchgeführt, weil damit eine Diazotierung in einem organischen Lösungsmittel wie beispielsweise Eisessig möglich ist, kein grosser Überschuss an Mineralsäure nötig und kein anorganisches Salz beteiligt ist. Selbstverständlich kann die Diazotierung auch nach der herkömmlichen Methode mit wässriger Salzsäure oder Schwefelsäure und Natriumnitrit durchgeführt werden, jedoch entfallen dann die genannten zusätzlichen Vorteile. Besonders bevorzugt ist die Diazotierung mit Nitrosylschwefelsäure in Essigsäure, welche nach Beendigung der Diazotierung mit Wasser verdünnt wird.

Das γ-Chloracetoacetylchlorid wird vorteilhaft in gelöster Form, beispielsweise in Form der bei der Herstellung nach CH-PS 642 611 anfallenden Dichlormethan-Lösung, eingesetzt. Bei der Kupplung mit dem Diazoniumsalz wird die Säurechloridgruppe von dem anwesenden Wasser zur Carbonsäuregruppe hydrolysiert und anschliessend decarboxyliert, so dass als Nebenprodukte HCl, $CO_2$ und die Säure des Diazoniumsalzes, also beispielsweise Schwefelsäure, entstehen.

Die Chlormethylgruppe des 3-Chlorpyruvaldehydarylhydrazons wird zweckmässig mit einem Thiolat umgesetzt. Als Thiolate eignen sich insbesondere Alkylthiolate oder Arylthiolate (Thiophenolate).

Die Thiolate werden vorteilhaft durch Deprotonierung der entsprechenden Thiole mit Basen erhalten. Für Thiole geringer Acidität werden dafür zweckmässig starke Basen, beispielsweise Alkalialkoholate in den entsprechenden Alkoholen als Lösungsmittel, eingesetzt, während für stärkere acide Thiole wie beispielsweise Thiophenole auch weniger starke Basen, beispielsweise Trialkylamine im polaren aprotischen Lösungsmitteln wie Acetonitril, verwendet werden können. Die Reaktion des Thiolats mit dem Chlorhydrazon wird vorzugsweise bei einer Temperatur von 0 bis 40 °C durchgeführt.

Das entsprechend substituierte Pyruvaldehydarylhydrazon wird anschliessend mit Hydroxylamin-O-sulfonsäure zu dem entsprechenden 4-substituierten 2-Aryl-2H-1,2,3-triazol cyclisiert. Die Cyclisierung von ähnlichen Hydrazonen mit Hydroxylamin-O-sulfonsäure ist aus der DE-OS 31 06 598 bekannt, die dort beschriebenen Reaktionsbedingungen sind auch für das erfindungsgemässe Verfahren geeignet.

Nach dem erfindungsgemässen Verfahren kann je nach dem eingesetzten Aryldiazoniumsalz bzw. dem zugrundeliegenden Arylamin und dem verwendeten Nucleophil eine grosse Zahl verschiedener Produkte hergestellt werden. Aus den bereits erwähnten Verbindungsklassen sind besonders bevorzugt: Diazoniumsalze des Anilins und substituierter Aniline, wie beispielsweise o-, m- und p-Toluidin, o-, m- und p-Chloranilin, o-, m- und p-Nitranilin, o-, m- und p-Anisidin, der verschiedenen isomeren Xylidine und Dichloraniline, seitenkettenhalogenierter Alkylaniline wie p-Trifluormethylanilin, gemischt substituierter Aniline wie Chlor- oder Nitrotoluidine oder Dichlor-trifluormethylaniline, insbesondere 2,6-Dichlor-4-trifluormethylanilin, oder Diazoniumsalze bi- und polycyclischer Amine wie beispielsweise Aminobiphenyl, $\alpha$- und $\beta$-Naphthylamin.

Als Nucleophile besonders bevorzugt sind Alkali-niederalkylthiolate, also die Alkalisalze der Niederalkylmercaptane, insbesondere mit Natrium als Alkalimetall, also beispielsweise Methanthiolat, Ethanthiolat, 1-Propanthiolat, 2-Propanthiolat und die Alkalisalze der verschiedenen isomeren Butylmercaptane, ganz besonders bevorzugt Natriummethanthiolat.

Die nachfolgenden Beispiele verdeutlichen die Durchführung des erfindungsgemässen Verfahrens.

Beispiel 1:

3-Chlorpyruvaldehyd-1-(2,6-dichlor-4-(trifluormethyl)phenyl)hydrazon

In 120 ml Essigsäure wurden 144,3 g 4-(Trifluormethyl)-2,6-dichloranilin (96%ig) gelöst und innerhalb von einer Stunde bei 20 °C mit 209,7 g Nitrosylschwefelsäure (40%ig in Schwefelsäuremonohydrat) versetzt.

Anschliessend wurde die Reaktionsmischung auf 0 °C abgekühlt und mit 480 ml Wasser verdünnt. Diese Lösung wurde bei -5 bis 0 °C innerhalb einer Stunde zu einer Lösung von 111,6 g $\gamma$-Chloracetoacetylchlorid in 400 ml Dichlormethan (hergestellt nach CH-PS 642 611) getropft.

Das Gemisch wurde noch 1 h bei 10 °C gerührt, anschliessend wurden die Phasen getrennt.

Die wässrige Phase wurde mit 100 ml Dichlormethan extrahiert, die vereinigten organischen Phasen eingedampft und der Rückstand mit 300 ml Cyclohexan aufgeschlämmt. Der ungelöste Feststoff wurde abfiltriert, zweimal mit je 100 ml Cyclohexan gewaschen und bei 40 Torr getrocknet.

Ausbeute: 183,6 g ockerfarbene Kristalle.

Schmp.: 105-108 °C

$^1$H-NMR ($CDCl_3$, 300 MHz) $\delta$: 4,68 (s,2H), 7,32 (s,1H), 7,65 (s,2H), 8,56 (br.s,1H)

Im MS(EI, 70eV):

m/z 332 ($M^+$, 35%, Isotopenverteilung entspr. 3Cl) 283 (16), 224 (100, 2Cl), 201 (24), 166 (40), 119 (22), 96 (41), 77 (25)

Beispiel 2:

3-(Methylthio)pyruvaldehyd-1-(2,6-dichlor-4-(trifluormethyl)phenyl)hydrazon

In 900 ml Methanol wurden 9,2 g Natrium gelöst. In die so erhaltene Natriummethylatlösung wurden 21,3 g Methanthiol eingeleitet. Anschliessend wurden bei 30 bis 40 °C portionsweise 110,0 g 3-Chlorpyruvaldehyd-1-(2,6-dichlor-4-(trifluormethyl)phenyl)hydrazon (hergestellt nach Beispiel 1) zugegeben. Das Reaktionsgemisch wurde noch 1 h bei 40 °C gerührt und anschliessend das Methanol im Wasserstrahlvakuum abdestilliert. Der Rückstand wurde mit 500 ml Wasser versetzt und dreimal mit je 250 ml Dichlormethan extrahiert. Die vereinigten Dichlormethan-Extrakte wurden über Natriumsulfat getrocknet und eingedampft.

Der Rückstand wurde in 200 ml n-Hexan aufgeschlämmt, abfiltriert und im Wasserstrahlvakuum getrocknet.
Ausbeute: 92,2 g (Gehalt (GC): 94,7%)
Schmp.: 88-90 °C

Beispiel 3:

2-(2,6-Dichlor-4-(trifluormethyl)phenyl)-4-(methylthiomethyl)-2H-1,2,3-triazol

In 75 ml Acetonitril/Wasser (4:1) wurden 5,25 g 3-(Methylthio)pyruvaldehyd-1-(2,6-dichlor-4-(trifluormethyl)phenyl)hydrazon (hergestellt nach Beispiel 2) gelöst und mit 2,36 g Hydroxylamin-O-sulfonsäure (93,4%ig) versetzt.
Das Reaktionsgemisch wurde 1 h bei 30 °C gerührt und anschliessend durch Zugabe von 20 ml gesättigter wässriger Natriumhydrogencarbonatlösung auf einen pH von 8 eingestellt und 5 h auf 70 °C erhitzt. Nach dem Abkühlen auf Raumtemperatur wurde das Acetonitril im Wasserstrahlvakuum abdestilliert und die zurückbleibende wässrige Lösung zweimal mit je 25 ml Dichlormethan extrahiert.
Die vereinigten Dichlormethanphasen wurden über Natriumsulfat getrocknet und eingedampft. Als Rückstand wurden 4,6 g Rohprodukt mit einem Gehalt (HPLC) von 63,4% erhalten. Zur Reinigung wurde das Rohprodukt aus 22 ml Isopropylalkohol heiss umkristallisiert.
Ausbeute: 2,3 g (Gehalt (HPLC): 93,3%)

## Patentansprüche

1. Verfahren zur Herstellung von 2-Aryl-2H-1,2,3-triazolen der allgemeinen Formel

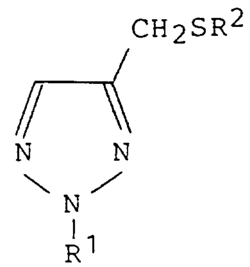

I

worin $R^1$ eine gegebenenfalls substituierte Aryl- oder Heteroarylgruppe und $R^2$ eine gegebenenfalls verzweigte und/oder substituierte offenkettige oder cyclische Alkylgruppe, oder eine gegebenenfalls substituierte Arylalkylgruppe, Arylgruppe oder Heteroarylgruppe bedeuten, dadurch gekennzeichnet, dass $\gamma$-Chloracetoacetylchlorid

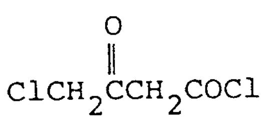

II

in einer ersten Stufe mit einem Diazoniumsalz der allgemeinen Formel

$$R^1N_2^+ \quad \frac{1}{n}A^{n-} \qquad \text{III}$$

worin $R^1$ die oben genannte Bedeutung hat und $A^{n-}$ ein Anion einer ein- oder mehrbasigen starken Säure ist, zu dem entsprechenden 3-Chlorpyruvaldehyd-arylhydrazon

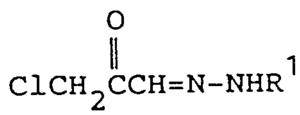

IV

gekuppelt, in einer zweiten Stufe mit einem Thiolat der allgemeinen Formel $\frac{1}{m}$ $Y^{m+}$ $R^2S^-$, worin $R^2$ die oben genannte Bedeutung hat und $Y^{m+}$ ein anorganisches oder organisches Kation ist, zu dem entsprechenden 3-substituierten Pyruvaldehyd-arylhydrazon

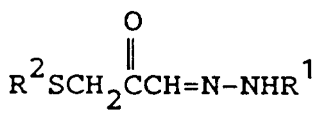

V

umgesetzt, und schliesslich in einer dritten Stufe mit Hydroxylamin-O-sulfonsäure zu der gewünschten Verbindung I cyclisiert wird.

**2.** Verfahren nach Patentanspruch 1, dadurch gekennzeichnet, dass ein Diazoniumsalz eingesetzt wird, worin $R^1$ eine gegebenenfalls substituierte Phenylgruppe ist.

**3.** Verfahren nach Patentanspruch 1 oder 2, dadurch gekennzeichnet, dass ein Diazoniumsalz eingesetzt wird, worin $A^{n-}$ Chlorid oder Hydrogensulfat ist.

**4.** Verfahren nach einem oder mehreren der Patentansprüche 1 bis 3, dadurch gekennzeichnet, dass das Diazoniumsalz durch Diazotierung des entsprechenden Amins mit Nitrosylschwefelsäure hergestellt wird.

**5.** Verfahren nach einem oder mehreren der Patentansprüche 1 bis 4, dadurch gekennzeichnet, dass als Thiolat ein Natriumalkylthiolat eingesetzt wird.

**6.** Verfahren nach einem oder mehreren der Patentansprüche 1 bis 5, dadurch gekennzeichnet, dass als Thiolat ein Methanthiolat eingesetzt wird.

**7.** Verfahren nach einem oder mehreren der Patentansprüche 1 bis 6, dadurch gekennzeichnet, dass die Umsetzung des 3-Chlorpyruvaldehydarylhydrazons mit dem Thiolat bei 0 bis 40 °C in einem protischen oder polaren aprotischen Lösungsmittel durchgeführt wird.

**8.** Verfahren nach einem oder mehreren der Patentansprüche 1 bis 7, dadurch gekennzeichnet, dass die Cyclisierung mit Hydroxylamin-O-sulfonsäure mit einem wässrig-organischen Lösungsmittelsystem durchgeführt wird, indem das substituierte Pyruvaldehydarylhydrazon zunächst bei einer Temperatur unter 40 °C mit der Sulfonsäure umgesetzt und dann mit einer Base der pH-Wert des Reaktionsgemisches auf einen Wert von 6-8 eingestellt und das Gemisch gegebenenfalls auf eine Temperatur bis 80 °C erhitzt wird.

**9.** Verfahren nach Patentanspruch 8, dadurch gekennzeichnet, dass als wässrig-organisches Lösungsmittelsystem ein Acetonitril-Wasser-Gemisch eingesetzt wird.

**10.** Verfahren nach einem oder mehreren der Patentansprüche 1 bis 9, dadurch gekennzeichnet, dass als Diazoniumsalz 2,6-Dichlor-4-trifluor-methylbenzoldiazoniumhydrogensulfat eingesetzt wird.

## Claims

**1.** A process for the preparation of 2-aryl-2H-1,2,3-triazoles of the general formula:

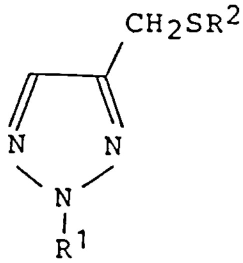

I

wherein $R^1$ is an optionally substituted aryl or heteroaryl group, and $R^2$ is an optionally branched and/or substituted, open-chained or cyclic alkyl group or an optionally substituted arylalkyl, aryl oder heteroaryl group,
characterized in that gamma-chloroacetoacetyl chloride:

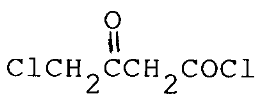

II

is coupled in a first step with a diazonium salt of the general formula:

$$R^1N_2^+ \quad \frac{1}{n}A^{n-} \qquad \text{III}$$

wherein $R^1$ has the above meaning, and $A^{n-}$ is the anion of a strong mono- or polybasic acid; to form the respective 3-chloropyruvaldehyde-aryl hydrazone:

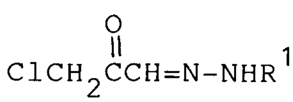

IV

is reacted in a second step with a thiolate of the general formula $\frac{1}{m}\,Y^{m+}\,R^2S^-$ wherein $R^2$ has the above meaning and $Y^{m+}$ is an inorganic or organic cation; to form the respective 3-substituted pyruvaldehyde-aryl hydrazone:

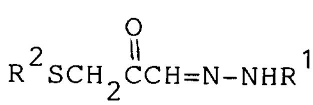

V

and is finally cyclized in a third step with hydroxylamine-O-sulfonic acid to form the desired compound I.

**2.** The process according to Claim 1, characterized in that a diazonium salt is used wherein $R^1$ is an optionally substituted phenyl group.

**3.** The process according to Claim 1 or 2, characterized in that a diazonium salt is used wherein $A^{n-}$ is chloride or hydrogen sulfate.

4. The process according to one or more of Claims 1 to 3, characterized in that the diazonium salt is prepared by diazotizing the respective amine with nitrosyl sulfuric acid.

5. The process according to one or more of Claims 1 to 4, characterized in that sodium alkylthiolate is used as a thiolate.

6. The process according to one or more of Claims 1 to 5, characterized in that a methane thiolate is used as a thiolate.

7. The process according to one or more of Claims 1 to 6, characterized in that the reaction of 3-chloropyruvaldehydearyl hydrazone with thiolate is conducted at 0 to 40 °C in a protic or polar aprotic solvent.

8. The process according to one or more of Claims 1 to 7, characterized in that cyclization with hydroxylamine-0-sulfonic acid is conducted with an aqueous-organic solvent system by first reacting the substituted pyruvaldehydearyl hydrazone with sulfonic acid at a temperature below 40 °C, then adjusting with a base the pH of the reaction mixture to a value of from 6 to 8, and optionally heating the mixture to a temperature up to 80 °C.

9. The process according to Claim 8, characterized in that a mixture of acetonitrile/water is used as an aqueous-organic solvent system.

10. The process according to one or more of Claims 1 to 9, characterized in that 2,6-dichloro-4-trifluoromethyl benzene diazonium hydrogensulfate is used as a diazonium salt.

**Revendications**

1. Procédé pour la préparation de 2-aryl-2H-1,2,3-triazoles de la formule générale

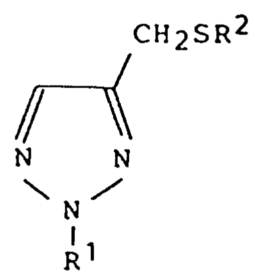

I

dans laquelle $R^1$ signifie un groupe aryle ou hétéroaryle, éventuellement substitué et $R^2$ signifie un groupe alkyle, éventuellement ramifié et/ou substitué à chaîne ouverte ou cyclique, ou un groupe arylalkyle, aryle ou hétéroaryle éventuellement substitué, caractérisé en ce que le chlorure de chloracétoacétyle

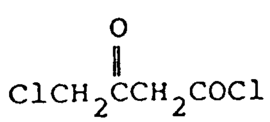

II

est mis en réaction dans une première étape avec un sel de diazonium de la formule générale

$$R^1N_2^+ \quad \frac{1}{n}A^{n-} \qquad III$$

dans laquelle $R^1$ a la signification précitée et $A^{n-}$ est un anion d'un acide fort mono- ou pluribasique lié à la 3-chloropyruvaldéhyd-arylhydrazone correspondante

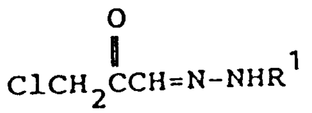

IV

dans une deuxième étape avec un thiolate de formule générale $\frac{1}{m}$ $Y^{m+}$ $R^2S^-$, dans laquelle $R^2$ a la signification précitée, et $Y^{m+}$ est un cation minéral ou organique, transformé en la pyruvaldéhyd-arylhydrazone correspondante substituée en 3

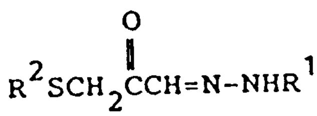

V

et pour finir cyclisé dans une troisième étape avec l'acide hydroxylamine-O-sulfonique en le composé I souhaité.

**2.** Procédé selon la revendication 1, caractérisé en ce que l'on met en oeuvre un sel de diazonium dans lequel $R^1$ est un groupe phényle éventuellement substitué.

**3.** Procédé selon la revendication 1 ou 2, caractérisé en ce que l'on met en oeuvre un sel de diazonium dans lequel $A^{n-}$ est un chlorure ou un sulfate d'hydrogène.

**4.** Procédé selon l'une ou plusieurs des revendications 1 à 3, caractérisé en ce que le sel de diazonium est préparé par diazotation de l'amine correspondante avec de l'acide nitrosylsulfurique.

**5.** Procédé selon l'une ou plusieurs des revendications 1 à 4, caractérisé en ce qu'en tant que thiolate, on met en oeuvre un alkylthiolate de sodium.

**6.** Procédé selon l'une ou plusieurs des revendications 1 à 5, caractérisé en ce qu'en tant que thiolate, on met en oeuvre un méthanethiolate.

**7.** Procédé selon l'une ou plusieurs des revendications 1 à 6, caractérisé en ce que la réaction de la 3-chloropyruvaldéhydarylhydrazone est conduite avec le thiolate à 0 jusqu'à 40 °C dans un solvant protique ou polaire aprotique.

**8.** Procédé selon l'une ou plusieurs des revendications 1 à 7, caractérisé en ce que la cyclisation est effectuée avec un acide hydroxylamine-O-sulfonique avec un système solvant aqueux organique, en ce que la pyruvaldéhydarylhydrazone substituée est d'abord mise en réaction à une température inférieure à 40 °C avec l'acide sulfonique, puis ajustée avec une base de la valeur de pH du mélange réactionnel à une valeur de 6-8 et en ce que le mélange est éventuellement chauffé à une température jusqu'à 80 °C.

**9.** Procédé selon la revendication 8, caractérisé en ce qu'en tant que système solvant aqueux organique, on met en oeuvre un mélange acétonitrile-eau.

**10.** Procédé selon l'une ou plusieurs des revendications 1 à 9, caractérisé en ce qu'en tant que sel de diazonium, on met en oeuvre du sulfate hydrogéné de 2,6-dichlor-4-trifluor-méthylbenzoldiazonium.